# FASCICULE DE BREVET EUROPEEN

(11) **EP 2 796 539 B1**
(45) Date de publication et mention de la délivrance du brevet: **16.09.2020**
(21) Numéro de dépôt: 14170428.8
(22) Date de dépôt: 24.07.2009
(51) Int. Cl.: C12M 1/34, G01N 27/22, G01N 33/487

(54) **Dispositif capteur à usage unique avec des électrodes realisées par depot métallique**
Einweg-Sensorvorrichtung mit Elektroden, die durch Metallablagerung ausgeführt werden
Disposable sensor device with electrodes manufactured by metal deposition

(30) Priorité: 25.07.2008 FR 0855138
(43) Date de publication de la demande: 29.10.2014
(62) Demande divisionnaire de: 09740390.1
(73) Titulaire: Hamilton Bonaduz AG, 7402 Bonaduz (CH)
(72) Inventeur: Ossart, Frédéric, 30980 Langlade (FR)
(74) Mandataire: Ruttensperger Lachnit Trossin Gomoll

(56) Documents cités:
- WO-A1-01/79828
- WO-A1-91/00903
- WO-A1-03/097861
- FR-A1- 2 867 278
- US-A1- 2005 176 155
- US-A1- 2005 272 146

## Description

La présente invention concerne des dispositifs capteurs de biomasse à usage unique. Elle vise également un procédé pour réaliser de tels capteurs, associés à des bioréacteurs à usage unique ou à une sonde/support en inox sur des bioréacteurs classiques, en inox et verre.

Un dispositif capteur selon le préambule de la revendication 1 est montré dans WO 01/79828 A1. De plus, US 2005/0272146 A1 divulgue la possibilité générale d'équiper des bioréacteurs à usage unique avec des capteurs ou détécteurs intégrants et de les mettre au rebut ensemble après usage.

L'élaboration de nouvelles molécules par voie biotechnologique est toujours considérée comme l'axe principal de développement pharmaceutique. Aujourd'hui, l'industrie pharmaceutique reconnaît des avantages notables associés à l'usage unique:
- la sécurité, du fait de la limitation du risque de contamination croisée,
- et les gains de productivité obtenus (i) par réduction des coûts de fonctionnement, notamment des frais de personnel; (ii) par mise en œuvre d'une culture à haute densité contribuant à limiter les temps de culture et l'occupation au sol; et (iii) par une mise en échelle industrielle plus rapide et moins coûteuse.

Traditionnellement les bio réacteurs mis en œuvre sont réutilisables, en verre ou en cuve inox, nécessitant leur nettoyage, stérilisation et re-validation constante. Ces opérations, de plus en plus coûteuses suite à la croissance continue des exigences réglementaires et de qualité, sont éliminées par l'implantation de solutions à usage unique.

Il existe une demande croissante pour des équipements tels que des bio réacteurs fermenteurs, cuve de stockage et préparation « upstream & downstream » (« flux montants & flux descendants ») à usage unique. Or de tels systèmes sont nécessairement éq uipés de capteurs prévus pour mesurer des paramètres physico-chimiques, biochimiques ou biophysiques. On peut notamment citer des capteurs réalisant une spectroscopie d'impédance, en particulier les capteurs de technologie capacitive conçus par le présent déposant.

Ces capteurs capacitifs permettent de détecter des variations relatives de capacités de 10^{"5} dans des milieux de faible résistance (quelques dizaines d'Ohms). Cette technique permet également, grâce à la spectroscopie d'impédance, de déterminer les paramètres des courbes de β dispersion (radio fréquence). Les mesures fournissent alors non seulement une concentration de cellules vivantes mais aussi des indications sur la morphologie et l'état physiologique des cellules. Les sondes de mesures usuelles sont de taille macroscopique et destinées à des cuves de fermentation en inox. Elles exploitent typiquement des configurations à 4 électrodes pour s'affranchir des effets de double couche de polarisation.

On peut notamment citer des capteurs capacitifs spécifiquement dédiés aux mesures de conductance, en particulier pour des applications en purification de type « downstream ». Ces capteurs de conductance peuvent présenter les mêmes géométries et structures d'électrode que des capteurs capacitifs permettant des mesures complètes d'impédance avec mesures de capacitance et de conductance.

On connaît aussi l'utilisation d'un connecteur aseptique qui permet de rentrer des capteurs solides dans un fermenteur jetable à condition d'être dans un environnement très protégé et sous hotte à flux laminaire. Cette solution est globalement plus coûteuse en raison du prix de revient et de fonctionnement des capteurs solides: décontamination, démontage, nettoyage, montage du capteur. Par ailleurs, les capteurs solides prévus pour équiper des bioréacteurs à usage unique doit être stérilisés avant chaque utilisation, ce qui ne limite pas en soi le risque de contamination lié à l'enchaînement de tâches nécessaires.

Le but de la présente invention est de remédier à ces inconvénients en proposant un bioréacteur ou conteneur à usage unique intégrant un capteur capacitif qui présente des performances suffisantes pour des applications en bioréacteur, tout en ayant un coût de réalisation et un coût de fonctionnement minimisés.

Cet objectif est atteint avec un bioréacteur ou conteneur à usage unique comprenant une enceinte prévue pour contenir un milieu biologique, et au moins un dispositif pour capter au moins une mesure d'impédance dans un processus physique, physico-chimique et/ou biologique dans ledit milieu, selon la revendication 14.

Suivant un autre aspect de l'invention, il est proposé un dispositif à usage unique pour capter au moins une mesure d'impédance dans un processus physique, physico-chimique et/ou biologique, comprenant des moyens pour réaliser au moins une mesure d'impédance dans un processus physique, physico-chimique et/ou biologique dans ledit milieu, selon la revendication 1.

En particulier, ce dispositif capacitif à usage unique peut être spécifiquement dédié à la mesure de biomasse vivante dans un milieu biologique, au moyen de la mesure de capacitance.

Le dispositif capteur à usage unique selon l'invention peut être associé en ligne à d'autres capteurs physiques, physico-chimiques ou biochimiques à usage unique tel que des capteurs de température, pH, concentration en oxygène dissous, concentration en CO₂ dissous, densité optique, pression, niveau, concentration en ammonium, en glycérol, taux de lactate et glucose, et plus généralement tout autre capteur à usage unique dans le but d'améliorer la connaissance et le contrôle d'une culture biologique.

Un capteur optique utilisant une pastille fluorescente à usage unique est une technique avantageuse de couplage et concerne la mesure en ligne de paramètres tels que le pH, la concentration en oxygène dissous et la mesure de la concentration en CO₂ dissous. Il peut ainsi intégrer plusieurs capteurs à usage unique sur un même support.

Ainsi avec l'invention, il devient possible de réaliser le premier système multicapteur à usage unique en ligne, biocompatible et stérilisable, mesurant les étapes clés d'une culture cellulaire, en vue de la piloter selon des critères et des objectifs quantitatifs et qualificatifs de production à toute échelle. Il permettra ég a lement de cou pler le bio-diagnostic et les microtechnologies pour la caractérisation de milieux biologiques divers.

Dans un mode particulier de réalisation, le dispositif capteur selon l'invention est agencé pour être installé dans une enveloppe stérile à applications multiples.

Le dispositif capteur selon l'invention peut aussi être agencé pour être inséré et plongeant dans une enceinte prévue pour contenir des cellules biologiques.

Dans une variante de l'invention, le dispositif capteur à usage unique selon l'invention est agencé pour être installé dans un bioréacteur réalisé de préférence pour une utilisation en lit fixe, c'est-à-dire tout type de culture de cellules sur support adhérent, micro-porteur et macro-porteur ou toute autre application dans laquelle les mesures recherchées sont issues d'un procédé de culture de type adhérent. Il peut par exemple être inséré dans un panier plongeant dans une enceinte prévue pour contenir des cellules biologiques dont le modèle de croissance est considéré statique, par exemple, pour la culture de cellules souches dans le but d'applications médicales.

Dans une autre variante de l'invention, le dispositif capteur à usage unique selon l'invention est agencé pour être installé dans un bioréacteur à usage unique.

Les moyens de fixation sont par exemple agencés pour permettre la fixation du capteur sur une paroi latérale ou sur le fond du bioréacteur à usage unique.

Le dispositif capteur selon l'invention peut aussi être inséré dans un passage aménagé dans une paroi du bioréacteur et présenter une face de captation orientée et en contact avec l'intérieur de ce bioréacteur.

Dans une forme de réalisation avantageuse, le dispositif capteur à usage unique comprend des électrodes s'étendant sur la partie de captation.

Il peut être réalisé sous la forme d'une pièce mécanique usinée agencée pour recevoir sur une partie de captation les électrodes et sur une partie de connexion des moyens de sortie de signal de mesure.

Il peut aussi être réalisé sous la forme d'une pièce moulée par injection incluant sur une partie de captation les électrodes et sur une partie de connexion les moyens de sortie de signal de mesure. Les moyens de sortie de signal de mesure peuvent comprendre un connecteur ou bien un câble ou un flexible.

Les électrodes, selon l'invention présente, sont réalisées par dépôt métallique sur un support isolant qui peut être un flexible.

Ce support flexible peut être réalisé sous la forme de deux films isolants dont au moins un supporte une électrode réalisée par dépôt et reliée électriquement à un conducteur disposé entre les deux films isolants.

Le dispositif capteur à usage unique peut être agencé pour être installé dans une enveloppe stérile à applications multiples.

Selon l'invention présente le dispositif capteur à usage unique comprend des moyens embarqués pour traiter localement des signaux de mesure d'impédance. Ces moyens embarqués peuvent être prévus pour produire un signal d'alerte en réponse à une détection d'anomalie par les moyens de traitement embarqués.

Suivant un autre aspect de l'invention, il est proposé un procédé pour réaliser un dispositif capteur à usage unique selon l'invention, mis en œuvre dans un environnement de production répondant aux critères d'asepsie et de stérilité requis pour l'utilisation d'un bioréacteur.

En effet, toutes les techniques mises en œuvre (soudure, lavage, nettoyage, décontamination bactérienne et virale, outils mécaniques, moyens d'analyse) représentent une méthodologie nouvelle de production qui jusque là n'avait pas était mise en œuvre pour la production de capteurs solides et traditionnels.

Les matériaux mis en œuvre pour la réalisation du dispositif capteur à usage unique sont nécessairement biocompatibles. Ces matériaux peuvent avantageusement être compatibles pour une stérilisation par gamma-irradiation avant et/ou après l'installation du dispositif capteur sur le bioréacteur.

La présente invention est aussi applicable à des procédés de mesure hors ligne tels que du diagnostic à usage médical, du contrôle qualité biologique, médical, environnemental, ou de l'emballage instrumenté spécifique pour le contrôle biologique, et n'est donc pas limitée aux seules enceintes stériles avec mesure en ligne.

Dans une autre application d'un capteur de biomasse à usage unique selon l'invention, celui-ci est monté sur une enceinte aseptique et est éventuellement associé à un capteur physique ou biologique, pour réaliser un conteneur de stockage de milieu biologique jetable sur lequel sont intégrés des capteurs jetables. Ces derniers permettent à tout moment de contrôler la biomasse vivante et d'autres paramètres physico-chimiques avec d'autres capteurs associés.

Ce moyen de stockage peut être réalisé à partir des mêmes capteurs et matériaux d'enceinte que pour l'application bio-reacteur. Il permet de détecter tout incident biologique pendant le stockage de la matière, sans risque de perte de stérilité pendant ce contrôle car il n'y pas de prise d'échantillon.

Cette application est particulièrement indiquée pour le contrôle industriel de matière biologique ou chimique avant et après livraison dans le cadre d'un suivi de qualité du produit. Elle procure également un avantage certain pour toute conservation de longue durée pour laquelle il est nécessaire d'avoir un contrôle régulier de la qualité ou d'estimer la péremption d'un produit tel que les denrées alimentaires.

Il devient ainsi possible de réaliser un emballage intelligent permettant de détecter, au moyen de ses capteurs intégrés, toute anomalie ou accident de conservation, et de procurer une traçabilité du transport de compositions, de produits ou de denrées impliquant des exigences de sécurité et de qualité.

D'autres avantages et caractéristiques de l'invention apparaîtront à l'examen de la description détaillée d'un mode de mise en œuvre nullement limitatif, et des dessins annexés sur lesquels :
- la figure 1 est une vue schématique d'un capteur à usage unique selon l'invention mis en œuvre dans un réacteur à usage unique en lit fixe ;
- La figure 2 est une vue schématique d'un bioréacteur à usage unique pour cultures de cellules en suspension équipé de capteurs à usage unique selon l'invention ;
- La figure 3 illustre un exemple pratique d'un bioréacteur à usage unique équipé de capteurs à usage unique selon l'invention ;
- La figure 4A est une vue en coupe d'un capteur à usage unique à quatre électrodes selon l'invention ;
- La figure 4B est une vue de dessus du capteur à usage unique de la figure 4A ;
- La figure 5A est une vue en coupe d'un premier mode de connexion des électrodes d'un capteur à usage unique selon l'invention réalisé à partir d'une pièce usinée et mettant en œuvre un connecteur enrobé d'une colle ;
- La figure 5B est une vue en coupe d'un second mode de connexion des électrodes d'un capteur à usage unique selon l'invention réalisé à partir d'unepièce usinée et mettant en œuvre un câble ou un flexible;
- La figure 6A est une vue en coupe d'un capteur à usage unique pourvu d'électrodes et d'un connecteur surmoulés par injection ;
- La figure 6B est une vue en coupe d'un capteur à usage unique pourvu d'électrodes et d'un câble ou flexible surmoulés par injection ;
- Les figures 7A, 7B et 7C illustrent un exemple particulier d'un capteur à usage unique selon l'invention dans lequel les électrodes sont déposées directement sur un flexible ;
- La figure 8 illustre une combinaison d'un capteur à usage unique de mesure d'impédance selon l'invention et d'un capteur à usage unique de mesure de fluorescence; dans un montage séparé, et
- La figure 9 illustre la combinaison d'un capteur à usage unique de mesure d'impédance selon l'invention et d'un capteur à usage unique de mesure de fluorescence, montés sur un même support.

On va maintenant décrire, en référence aux figures précitées, plusieurs exemples de capteurs à usage unique selon l'invention, dans différentes configurations de mise en œuvre.

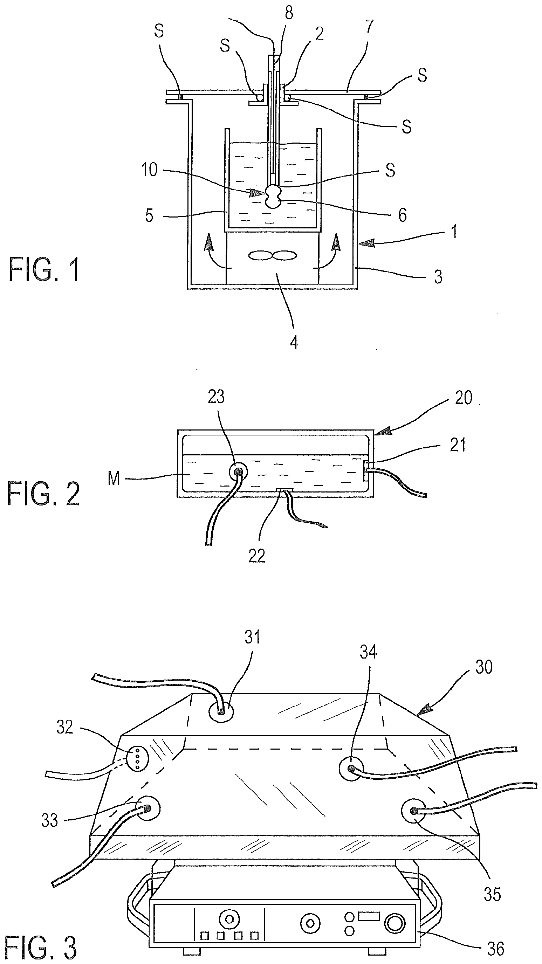

On va en premier lieu décrire un exemple de mise en œuvre d'un capteur d'impédance à usage unique 10 mis en œuvre dans un bioréacteur réutilisable 1, en référence à la figure 1. Ce bioréacteur 1 peut, à titre d'exemple non limitatif, être du type de ceux commercialisés par les sociétés WAVE, SARTORIUS ou ATMI.

Le capteur d'impédance mis en œuvre peut être du type capacitif tel que celui commercialisé par le présent déposant et divulgué dans le document FR2 812 725. Il peut aussi être du type « multicapteur », comme ceux incluant des capteurs optiques qui sont divulgués dans le document FR2 867 279 au nom du présent déposant.

Le bioréacteur 1 comprend, dans une enceinte 3, un lit fixe 5 contenant des cellules biologiques et équipé d'un agitateur 4, un couvercle 7 fixé à l'enceinte 3 par des soudures D et percé d'un orifice 2 dans lequel est inséré un tube récepteur 9 prévu pour recevoir un capteur d'impédance à usage unique 10 pourvu à son extrémité d'un jeu d'électrodes 6. Ces électrodes peuvent être au nombre de deux, quatre ou plus, en fonction de la technique de mesure mise en œuvre. Le capteur d'impédance 10 comporte des conducteurs 8 pour délivrer les signaux de mesure générés par ce capteur.

Lorsqu' il s'agit d'équiper un bioréacteur à usage unique 20, en référence à la figure 2, des capteurs à usage unique 21, 22, 23 peuvent être installés respectivement sur une face latérale, le fond et la face avant de la cuve de ce bioréacteur. Ainsi, comme l'illustre la figure 3, un bioréacteur à usage unique 30 mis en œuvre sur un appareil de contrôle 36 à usage permanent peut être fourni entièrement équipé de capteurs à usage unique 31-35.

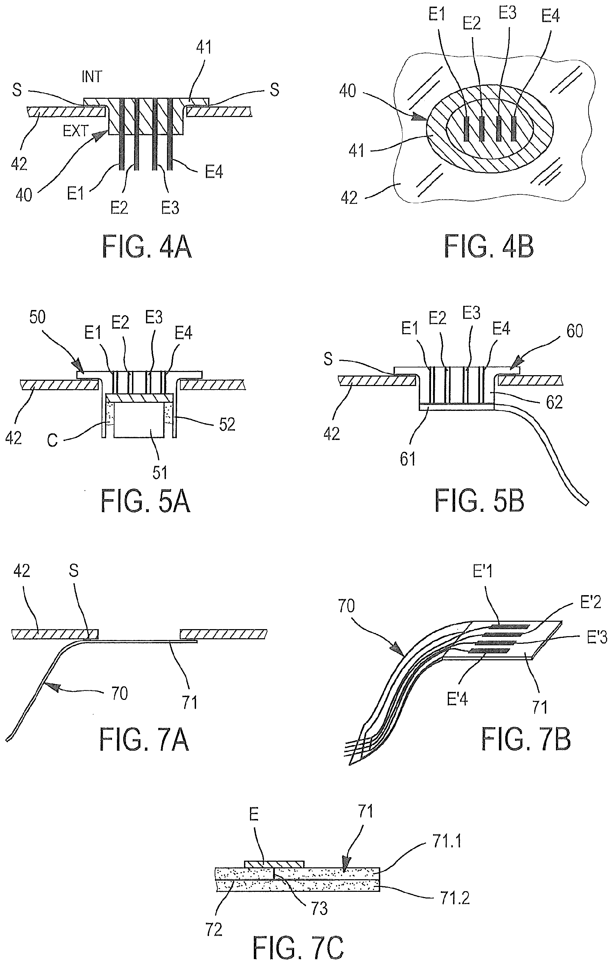

En référence aux figures 4A et 4B, un capteur de mesure d'impédance unique 40 peut être disposé dans un orifice d'une paroi 42 d'un bioréacteur, avec une partie de captation dont une face de captation 41 est disposée à l'intérieur du bioréacteur et fixée par soudure S à la face intérieure de cette paroi. Le capteur de mesure d'impédance fait alors partie intégrante du bioréacteur.

On peut aussi prévoir d'autres modes de fixation tels que le vissage, l'emmanchage ou le collage. Le capteur 40 est pourvu sur la face de captation 41 de quatre électrodes E1-E4, dont deux assurent une fonction d'excitation et deux une fonction de mesure.

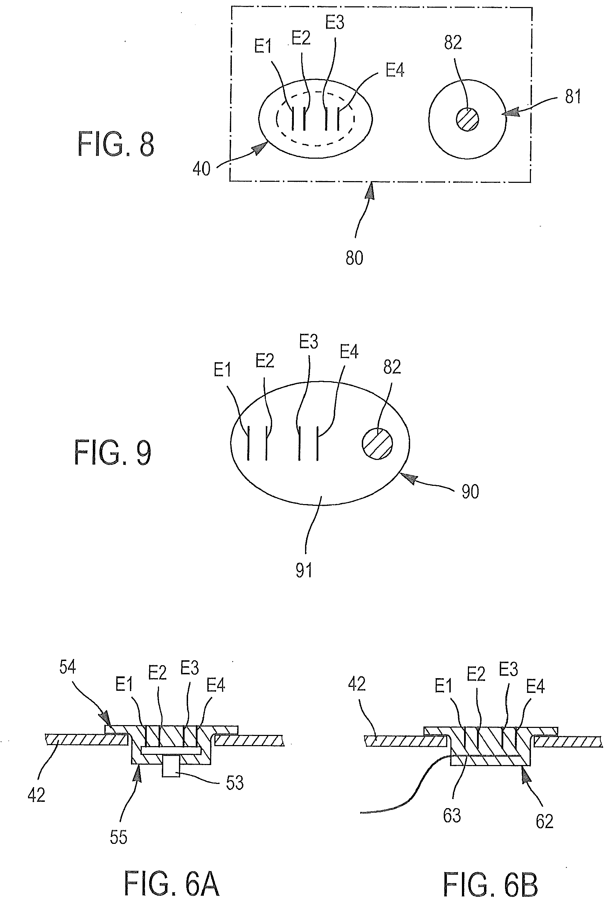

La connexion des électrodes d'un capteur à usage unique à un appareil de mesure ou de contrôle peut être effectuée, soit au moyen d'un connecteur (figures 5A et 6A), soit au moyen d'un câble ou flexible (figures 5B et 6B). Ces moyens de connexion constituent la partie de connexion du capteur à usage unique.

Le capteur à usage unique peut être réalisé soit à partir d'une pièce en matière plastique usinée (figures 5A et 5B), soit par injection avec surmoulage des électrodes et des moyens de connexion (figures 6A et 6B).

Les électrodes d'un capteur de mesure d'impédance à usage unique peuvent être réalisées :
- soit sous la forme de pièces métalliques qui peuvent être collées (figures 4A, 4B, 5A, et 5B) ou surmoulées (figures 6A et 6B),
- soit, comme choisi selon l'invention présente, par déposition, par exemple par des techniques de dépôt sous vide, par sérigraphie, lithographie, ou électrolyse.

Dans une première configuration illustrée par la figure 5A, le capteur à usage unique 50 inséré dans un orifice d'un bioréacteur est réalisé à partir d'une pièce usinée 52 dans laquelle ont été disposés quatre électrodes E1-E4 et un connecteur 51 fixé à la pièce usinée par collage C.

Dans une seconde configuration illustrée par la figure 5B, le capteur à usage unique 60, réalisé à partir d'une pièce usinée 62, est équipé d'un flexible ou d'un câble 61 relié électriquement aux quatre électrodes E1-E4.

Dans une troisième configuration illustrée par la figure 6A, le capteur à usage unique 55 est réalisé par injection de matière plastique avec surmoulage des quatre électrodes E1-E4 et d'un connecteur 53.

Dans une quatrième configuration, le capteur à usage unique 62 est réalisé par injection de matière plastique avec surmoulage des quatre électrodes E1-E4 et d'un flexible 63.

Dans un autre mode de réalisation choisi selon l'invention présente et illustré par les figures 7A, 7B et 7C, les électrodes E'I-E'4 d'un capteur à usage unique 70 sont réalisées par dépôt métallique directement sur un flexible 71. Ce capteur 70 peut ensuite être fixé par soudure S sur la paroi 42 d'un bioréacteur.

En référence à la figure 7C, le flexible 71 est réalisé par superposition de deux flexibles isolants 71.1, 71.2 entre lesquels est réalisée une piste conductrice 72 reliée à une électrode déposée E au moyen d'un via 73 traversant le flexible isolant 71.1 sur la face extérieure duquel est déposée l'électrode E.

Un capteur de mesure d'impédance à usage unique 40 peut être combiné à un autre capteur à usage unique, par un exemple un capteur de fluorescence pour la mesure du pH, de l'oxygène (O₂) ou du gaz carbonique (CO₂).

Les deux capteurs à usage unique peuvent être soit montés séparément (figure 8), soit disposés sur un même support (figure 9). Dans le premier mode, chaque capteur à usage unique 40, 82 est fourni sur un support distinct 41, 81, tandis que dans le second mode, les éléments actifs des deux capteurs - les quatre électrodes E1-E4 du capteur de mesure d'impédance, et le capteur de fluorescence 82 - sont disposés sur un même patch ou support 91 et constituent un double capteur à usage unique 90.

On va maintenant décrire des contraintes de réalisation de capteurs à usage unique selon l'invention. Selon les règles de bonnes pratiques de production (BPF ou cGMP), les solvants et toutes pollutions sont à proscrire des procédés de fabrication pour éviter de polluer les bio-matériaux utilisés dans la réalisation industrielle du capteur. En particulier, la norme "leachables and extractables" (« relargables et extractibles ») (21 CFR part 600.11) doit être suivie.

La chaîne de stérilisation ne doit pas être rompue entre la fabrication d'un capteur et son implantation chez un sous-traitant. Les capteurs à usage unique sont envoyés dans un conditionnement stérile et sont montés sous un flux laminaire sur la poche. Une fois que tous les éléments sont montés, la poche est stérilisée par rayonnement gamma.

Les matériaux utilisés doivent bien sûr être bio-compatibles et résistants à la dose de rayon gamma mise en oeuvre
Le capteur à usage unique doit être pré-décontaminé, i .e. être débarrassé de tout polluant sous un seuil accepté par l'application. Ces polluants sont principalement capturés lors des processus utilisés pour la fabrication du capteur (usinage, montage, soudage, collage...). L'emballage des capteurs à usage unique est étudié pour conserver la stérilité du capteur et pour être compatible aux exigences et contraintes pour une utilisation en salle propre. Ainsi, un emballage à double parois est préconisé.

Le capteur est monté sur le bio-reacteur et la stérilisation gamma est effectuée préférentiellement après le montage du capteur et de tous les constituants (tuyaux, bouchons, raccords,...). Pour d'autres applications, la stérilisation pourra être réalisée juste après l'emballage du capteur. D'autres moyens de stérilisation peuvent être également envisagés.

Les capteurs à usage unique peuvent être connectés, par voie filaire ou non filaire, à des équipements électroniques de mesure d'impédance disponibles sur le marché ou à des équipements plus spécifiques à la mesure de biomasse tel que celui divulgué dans le document FR2 812 725.

Selon l'invention présente il est aussi prévu que tout ou partie de l'électronique de mesure et de traitement soit embarquée sur le bioréacteur, l'emballage ou le conteneur intelligent, en utilisant des technologies d'intégration, éventuellement avec autoalimentation électrique. En particulier, des dispositifs d'alerte, optiques ou sonores, peuvent être aussi associés aux capteurs à usage unique selon l'invention. Par exemple dans le cas d'un emballage intelligent prévu pour stocker un milieu biologique, un signal d'alerte peut être généré lorsqu'une évolution anormale de la capacitance ou d'une grandeur physique ou physico-chimique est détectée. On peut notamment se référer au document FR2 874 264 au nom du présent déposant, qui divulgue un procédé et dispositif de détermination de biomasse dans un milieu.

Des techniques de fabrication issues de l'électronique et de la microélectronique pourraient être mises en œuvre pour une fabrication à grande échelle d'électrodes d'un capteur à usage unique selon l'invention. En particulier, un circuit intégré de détection capacitive pourrait être réalisé par des techniques de diffusion, par exemple sur des substrats polymères ou des substrats couramment employés pour la réalisation de microsystèmes

Bien sûr, l'invention n'est pas limitée aux exemples qui viennent d'être décrits et de nombreux aménagements peuvent être apportés à ces exemples sans sortir du cadre de l'invention.

## Revendications

1. Dispositif capteur à usage unique (10; 21-23; 31-35; 40, 50, 60, 70, 90) pour capter au moins une mesure d'impédance dans un processus physique, physico-chimique et/ou biologique (1, 20, 30), comprenant des moyens pour réaliser au moins une mesure d'impédance dans un processus physique, physico-chimique et/ou biologique dans un milieu biologique contenue dans un enceinte, ce dispositif comprenant une partie de captation en contact direct avec ledit milieu et une partie de connexion s'étendant vers l'extérieur de ladite enceinte, ce dispositif comprenant des électrodes (E1-E4) s'étendant sur la partie de captation, et étant réalisé sous la forme d'une pièce mécanique usinée (52, 62) agencée pour recevoir sur ladite partie de captation les électrodes (E1-E4) et sur ladite partie de connexion des moyens de sortie de signal de mesure (51, 61), les électrodes (E'1-E'4) étant réalisées par dépôt métallique sur un support isolant (71),
**caractérisé en ce que** ce dispositif est agencé pour être fixé au travers d'une paroi de l'enceinte prévue pour contenir le milieu biologique, de façon à en être partie intégrante et que ce dispositif comprend en outre des moyens embarqués pour traiter localement des signaux de mesure d'impédance.

2. Dispositif capteur à usage unique (70) selon la revendication 1,
**caractérisé en ce que** le support (71) de dépôt des électrodes est un flexible.

3. Dispositif capteur à usage unique (70) selon la revendication 1 ou 2,
**caractérisé en ce que** le support flexible (71) est réalisé sous la forme de deux films isolants (71.1, 71.2) dont au moins un supporte une électrode (E) réalisée par dépôt et reliée électriquement à un conducteur (72) disposé entre les deux films isolants (71.1, 71.2).

4. Dispositif capteur à usage unique (40, 90) selon l'une quelconque des revendications précédentes,
**caractérisé en ce qu'**il est associé à au moins un autre capteur physique, physico-chimique ou biochimique à usage unique (82).

5. Dispositif capteur à usage unique (55, 62) selon l'une quelconque des revendications précédentes,
**caractérisé en ce qu'**il est réalisé sous la forme d'une pièce moulée par injection incluant sur une partie de captation les électrodes (E1-E4) et sur une partie de connexion les moyens de sortie de signal de mesure (53, 63).

6. Dispositif capteur à usage unique (50, 55) selon l'une quelconque des revendications précédentes,
**caractérisé en ce que** les moyens de sortie de signal de mesure comprennent un connecteur (51, 53).

7. Dispositif capteur à usage unique (60, 62) selon l'une quelconque des revendications précédentes,
**caractérisé en ce que** les moyens de sortie de signal de mesure comprennent un câble ou un flexible (61, 63).

8. Dispositif capteur à usage unique (40, 50, 60, 55, 62) selon l'une quelconque des revendications précédentes,
**caractérisé en ce que** des électrodes (E1-E4) sont réalisées à partir de pièces métalliques.

9. Dispositif capteur à usage unique (10) selon l'une quelconque des revendications précédentes,
**caractérisé en ce qu'**il est agencé pour être installé dans une enveloppe stérile à applications multiples (1).

10. Dispositif capteur à usage unique (90) selon l'une quelconque des revendications précédentes et la revendication 4,
**caractérisé en ce qu'**il intègre le capteur physique, physico-chimique ou biochimique additionnel à usage unique (82) sur un même support (91).

11. Dispositif capteur à usage unique selon l'une quelconque des revendications précédentes,
**caractérisé en ce qu'**il comprend en outre des moyens embarqués pour produire un signal d'alerte en réponse à une détection d'anomalie par les moyens de traitement embarqués.

12. Dispositif capteur à usage unique selon l'une quelconque des revendications précédentes,
**caractérisé en ce qu'**il est agencé pour être fixé par soudure sur une paroi, en particulier sur une face externe de la paroi, d'un bioreacteur.

13. Procédé pour réaliser un dispositif capteur à usage unique selon l'une des revendications précédentes, ledit procédé étant mis en oeuvre dans un environnement de production répondant aux critères d'asepsie et de stérilité requis pour l'utilisation d'un bioréacteur, et comprenant une stérilisation du dispositif capteur à usage unique après son installation sur un bioréacteur.

14. Bioréacteur ou conteneur à usage unique (20, 30) comprenant une enceinte prévue pour contenir un milieu biologique, et au moins un dispositif capteur pour capter au moins une mesure d'impédance dans un processus physique, physico-chimique et/ou biologique dans ledit milieu, ce dispositif étant fixé au travers d'une paroi de ladite enceinte de façon à en être partie intégrante et comprend une partie de captation en contact direct avec l'intérieur dudit bioréacteur ou conteneur,
**caractérisé en ce que** ce dispositif capteur est un dispositif selon l'une quelconque des revendications précédentes, qui comprend une partie de connexion s'étendant vers l'extérieur de ladite enceinte.

## Patentansprüche

1. Einweg-Sensorvorrichtung (10; 21-23; 31-35; 40, 50, 60, 70, 90) zum Erfassen wenigstens einer Impedanzmessung in einem physikalischen, physikalisch-chemischen oder/und biologischen Verfahren (1, 20, 30), umfassend Mittel zum Durchführen wenigstens einer Impedanzmessung in einem physikalischen, physikalisch-chemischen oder/und biologischen Verfahren in einem in einer Einfassung enthaltenen biologischen Milieu, wobei die Vorrichtung einen Erfassungsabschnitt umfasst, welcher in unmittelbarem Kontakt mit dem biologischen Milieu steht, und einen Verbindungsabschnitt umfasst, welcher sich zur Außenumgebung der Einfassung erstreckt, wobei die Vorrichtung Elektroden (E1-E4) umfasst, welches sich auf dem Erfassungsabschnitt erstrecken, und wobei die Vorrichtung in Gestalt eines bearbeiteten mechanischen Teils (52, 62) realisiert ist, welches dazu ausgebildet ist, auf dem Erfassungsabschnitt die Elektroden (E1-E4) aufzunehmen und auf dem Verbindungsabschnitt Mittel zur Ausgabe eines Messsignals (51,61) aufzunehmen, wobei die Elektroden (E'1-E'4) durch metallische Abscheidung auf einem isolierenden Träger (71) realisiert sind,
**dadurch gekennzeichnet, dass** die Vorrichtung dazu ausgebildet ist, mittels einer Wand der zur Aufnahme des biologischen Milieus vorgesehenen Einfassung derart befestigt zu werden, dass sie integraler Abschnitt derselben ist, sowie dadurch, dass die Vorrichtung darüber hinaus eingebaute Mittel umfasst, um Impedanzmesssignale lokal zu verarbeiten.

2. Einweg-Sensorvorrichtung (70) nach Anspruch 1,
**dadurch gekennzeichnet, dass** der Träger (71) der Elektrodenabscheidung ein flexibles Element ist.

3. Einweg-Sensorvorrichtung (70) nach einem der Ansprüche 1 oder 2,
**dadurch gekennzeichnet, dass** der flexible Träger (71) realisiert ist als zwei isolierende Folien (71.1, 71.2), von welchen wenigstens eine eine Elektrode (E) trägt, die durch Abscheidung realisiert ist und elektrisch mit einem Leiter (72) verbunden ist, welcher zwischen den zwei isolierenden Folien (71.1, 71.2) angeordnet ist.

4. Einweg-Sensorvorrichtung (40, 90) nach einem der vorhergehenden Ansprüche,
**dadurch gekennzeichnet, dass** sie wenigstens einem weiteren physikalischen, physikalisch-chemischen oder biochemischen Einweg-Sensor (82) zugeordnet ist.

5. Einweg-Sensorvorrichtung (55, 62) nach einem der vorhergehenden Ansprüche,
**dadurch gekennzeichnet, dass** sie als Spritzgussteil ausgebildet ist, welches an einem Erfassungsabschnitt die Elektroden (E1 - E4) und an einem Verbindungsabschnitt die Mittel zur Messsignalausgabe (53, 63) enthält.

6. Einweg-Sensorvorrichtung (50, 55) nach einem der vorhergehenden Ansprüche,
**dadurch gekennzeichnet, dass** die Mittel zur Messsignalausgabe einen Verbinder (51, 53) umfassen.

7. Einweg-Sensorvorrichtung (60, 62) nach einem der vorhergehenden Ansprüche,
**dadurch gekennzeichnet, dass** die Mittel zur Messsignalausgabe ein Kabel oder ein flexibles Element (61, 63) umfassen.

8. Einweg-Sensorvorrichtung (40, 50, 60, 55, 62) nach einem der vorhergehenden Ansprüche,
**dadurch gekennzeichnet, dass** die Elektroden (E1 - E4) ausgehend von Metallteilen realisiert sind.

9. Einweg-Sensorvorrichtung (10) nach einem der vorhergehenden Ansprüche,
**dadurch gekennzeichnet, dass** sie dazu konfiguriert ist, in einer sterilen Hülle für multiple Anwendungen (1) installiert zu werden.

10. Einweg-Sensorvorrichtung (90) nach einem der vorhergehenden Ansprüche und nach Anspruch 4,
**dadurch gekennzeichnet, dass** sie den zusätzlichen physikalischen, physikalisch-chemischen oder biochemischen Einweg-Sensor (82) auf einem gleichen Träger (91) integriert.

11. Einweg-Sensorvorrichtung nach einem der vorhergehenden Ansprüche,
**dadurch gekennzeichnet, dass** sie außerdem eingebaute Mittel umfasst, um ein Alarmsignal in Antwort auf eine Erfassung einer Anomalie durch die eingebauten Verarbeitungsmittel auszugeben.

12. Einweg-Sensorvorrichtung nach einem der vorhergehenden Ansprüche,
**dadurch gekennzeichnet, dass** sie dazu konfiguriert ist, durch Verschweißen auf einer Wand, insbesondere auf einer Außenfläche der Wand, eines Bioreaktors fixiert zu sein.

13. Verfahren zur Realisierung einer Einweg-Sensorvorrichtung nach einem der vorhergehenden Ansprüche, wobei das Verfahren in einer Herstellungsumgebung nach Maßgabe von Standards der Keimfreiheit und der Sterilität ausgeführt wird, welche zur Verwendung in einem Bioreaktor erforderlich sind, und umfassend eine Sterilisierung der Einweg-Sensorvorrichtung nach ihrer Installation an einem Bioreaktor.

14. Einweg-Bioreaktor oder Einweg-Behälter (20, 30), umfassend eine Einfassung, welche dazu vorgesehen ist, ein biologisches Milieu zu enthalten, und umfassend wenigstens eine Sensorvorrichtung, um wenigstens eine Impedanzmessung in einem physikalischen, physikalisch-chemischen oder/und biologischen Prozess in diesem Milieu zu erfassen, wobei die Sensorvorrichtung mittels einer Wand der Einfassung derart befestigt ist, dass sie einen integralen Bestandteil derselben bildet, und einen Erfassungsabschnitt umfasst, welcher sich in unmittelbarem Kontakt mit dem Innenraum des Bioreaktors oder Behälters befindet,
**dadurch gekennzeichnet, dass** die Sensorvorrichtung eine Vorrichtung nach einem der vorhergehenden Ansprüche ist, welche einen Verbindungsabschnitt aufweist, welcher sich zur Außenumgebung der Einfassung hin erstreckt.

## Claims

1. A single-use sensor device (10; 21-23; 31-35; 40, 50, 60, 70, 90) for sensing at least one impedance measurement in a physical, physicochemical, and/or biological process (1, 20, 30), comprising means for performing at least one impedance measurement in a physical, physicochemical, and/or biological process in a biological medium contained in an enclosure, the device comprising a sensing portion in direct contact with said medium and a connecting portion extending toward the exterior of said enclosure, said device comprising electrodes (E1 to E4) that extend on the sensing portion and being produced in the form of a machined mechanical part (52, 62) configured to receive the electrodes (E1 to E4) on said sensing portion and measured-signal output means (51, 61) on said connecting portion, the electrodes (E'1 to E'4) being produced by deposition of metal onto a flexible insulating support (71),
**characterized in that** said device is configured to be fastened through a wall of an enclosure provided for containing the biological medium so as to be an integral part thereof; and said device furthermore comprises onboard means for locally processing the measured impedance signals.

2. The single-use sensor device (70) according to Claim 1,
**characterized in that** the deposition support (71) of the electrodes is a flexible element.

3. The single-use sensor device (70) according to Claim 1 or 2,
**characterized in that** the flexible support (71) is produced in the form of two insulating films (71.1, 71.2), at least one of which supports an electrode (E) that is produced by deposition and is connected electrically to a conductor (72) arranged between the two insulating films (71.1, 71.2).

4. The single-use sensor device (40, 90) according to any one of the preceding claims,
**characterized in that** it is associated with at least one other single-use physical, physicochemical, or biochemical sensor (82).

5. The single-use sensor device (55, 62) according to any one of the preceding claims,
**characterized in that** it is produced in the form of an injection-molded part including the electrodes (E1 to E4) on a sensing portion and the measured-signal output means (53, 63) on a connecting portion.

6. The single-use sensor device (50, 55) according to any one of the preceding claims,
**characterized in that** the measured-signal output means comprise a connector (51, 53).

7. The single-use sensor device (60, 62) according to any one of the preceding claims,
**characterized in that** the measured-signal output means comprise a cable or a flexible element (61, 63).

8. The single-use sensor device (40, 50, 60, 55, 62) according to any one of the preceding claims,
**characterized in that** electrodes (E1 to E4) are produced from metal parts.

9. The single-use sensor device (10) according to any one of the preceding claims,
**characterized in that** it is configured to be installed in a sterile casing (1) having multiple applications.

10. The single-use sensor device (90) according to any one of the preceding claims and Claim 4,
**characterized in that** it incorporates the additional single-use physical, physicochemical, or biochemical sensor (82) on a single support (91).

11. The single-use sensor device according to any one of the preceding claims,
**characterized in that** it furthermore comprises onboard means for generating an alert signal in response to detection of an anomaly by the onboard processing means.

12. The single-use sensor device according to any one of the preceding claims,
**characterized in that** it is configured to be fastened by welding onto a wall, in particular onto an outer surface of the wall, of a bioreactor.

13. A method for producing a single-use sensor device according to one of the preceding claims, said method being implemented in a production environment meeting the asepsis and sterility criteria required for use of a bioreactor, and comprising sterilization of the single-use sensor device after installation thereof on a bioreactor.

14. A single-use bioreactor or container (20, 30) comprising: an enclosure provided for containing a biological medium; and at least one sensor device for sensing at least one impedance measurement in a physical, physicochemical, and/or biological process in said medium, said device being fastened through a wall of said enclosure so as to be an integral part thereof, and comprising a sensing portion in direct contact with the interior of said bioreactor or container,
**characterized in that** said sensor device is a device according to any one of the preceding claims which comprises a connecting portion extending toward the exterior of said enclosure.
